# EUROPEAN PATENT SPECIFICATION

(11) **EP 2 804 739 B1**
(45) Date of publication and mention of the grant of the patent: **02.01.2019**
(21) Application number: 12798998.6
(22) Date of filing: 26.11.2012
(51) Int. Cl.: B29C 55/00, C08J 5/18, D04H 1/4391, B01D 67/00

(54) **ARTICLES INCLUDING EXPANDED POLYTETRAFLUOROETHYLENE MEMBRANES WITH SERPENTINE FIBRILS**
ARTIKEL MIT EXPANDIERTEN POLYTETRAFLUORETHYLENMEMBRANEN MIT SCHLANGENFÖRMIGEN FIBRILLEN
ARTICLES INCLUANT DES MEMBRANES EN POLYTÉTRAFLUOROÉTHYLÈNE EXPANSÉ COMPORTANT DES FIBRILLES ONDULÉES

(30) Priority: 16.01.2012 US 201213351052; 13.11.2012 US 201213675730
(43) Date of publication of application: 26.11.2014
(73) Proprietor: W. L. Gore & Associates, Inc., Newark, DE 19711 (US)
(72) Inventor: WHITE, Charles, F., Newark DE 19711 (US); RADSPINNER, Rachel, Flagstaff, AZ 86004 (US)
(74) Representative: HGF Limited
(86) International application number: PCT/US2012/066518
(87) International publication number: WO 2013/109343

(56) References cited:
- EP-A2- 0 293 090
- WO-A1-95/05555
- WO-A1-97/10871
- US-A- 5 071 609
- US-A1- 2002 198 588
- US-A1- 2010 159 171

## Description

### FIELD OF THE INVENTION

The present invention relates to expanded polytetrafluoroethylene (ePTFE) membranes with serpentine fibrils and to high elongation materials made therefrom.

### DEFINITIONS

As used herein, the term "serpentine fibrils" means multiple fibrils that curve or turn one way then another.

As used herein, the term "controlled retraction" refers to causing articles to shorten in length in at least one direction by the application of heat, by wetting with a solvent, or by any other suitable means or combinations thereof in such a way as to inhibit folding, pleating, or wrinkling of the subsequent article visible to the naked eye.

The term "imbibed or imbibing" as used herein is meant to describe any means for at least partially filling at least a portion of the pores of a porous material such as ePTFE or the like.

The term "elongation" as used herein is meant to denote the increase in length in response to the application of a tensile force.

### BACKGROUND OF THE INVENTION

Porous fluoropolymer materials, and in particular, expanded polytetrafluoroethylene (ePTFE) materials, typically exhibit relatively low elongation when stressed in the direction parallel to the orientation of the fibrils. High strength expanded ePTFE materials have relatively low elongation values compared to lower strength expanded ePTFE materials. Uniaxially expanded materials can exhibit high elongation when stressed in the direction orthogonal to the fibrils, however, the membranes are exceptionally weak in this direction.

Uniaxially expanded ePTFE tubes positioned on mandrels have been mechanically compressed and heat treated to achieve higher elongations prior to rupture. Such tubes also exhibit recovery if elongated prior to rupture and released from stress. U.S. Patent No. 4,877,661 to House, et al. discloses porous PTFE having the property of rapid recovery and a method for producing these materials. Further, the pores of compressed tubes have been penetrated with elastomeric materials. For example, U.S. Patent No. 7,789,908 to Sowinski, et al. discloses an elastomeric recoverable PTFE material that includes longitudinally compressed fibrils of an ePTFE material penetrated by an elastomeric material within the pores which define an elastomeric matrix. U.S. patent application no. 2002/198588 to Joseph Armstrong et al. describes the use of ePTFE films to form articles such as a covered endoprosthesis.

A need continues to exist for thin, strong membranes that exhibit high degrees of elongation, such as greater than 50% elongation. Some applications further demand qualities such as thinness, low density, and/or small pore size, and combinations thereof. Other applications require a relatively low force to elongate the membrane.

### SUMMARY OF THE INVENTION

The present disclosure is directed to fluoropolymer membranes that exhibit high elongation while substantially retaining the strength properties of the fluoropolymer membrane. Such membranes characteristically possess serpentine fibrils.

According to the present invention, an article is provided that includes a retracted expanded fluoropolymer membrane that includes serpentine fibrils, the serpentine fibrils curving in a first direction and the a second direction, the serpentine fibrils being produced by the process comprising:
a. expanding a dried, extruded fluoropolymer tape in at least one direction to produce an initial expanded fluoropolymer membrane; and either
b. heating the initial expanded fluoropolymer membrane to thermally retract the expanded fluoropolymer membrane in at last one direction of expansion, or
c. adding a solvent to the initial expanded fluoropolymer membrane to retract the expanded fluoropolymer membrane in at least one direction of expansion.

The application of a tensile force at least partially straightens the serpentine fibrils, thereby elongating the article. In addition, the article has an elongation in at least one direction of at least about 50% and a matrix tensile strength of at least about 50 MPa. In some embodiments, the expanded fluoropolymer membrane includes a microstructure of substantially only fibrils. The expanded fluoropolymer membrane may have a matrix tensile strength in at least one direction of at least about 200 MPa.

In some embodiments the dried, extruded fluoropolymer tape is expanded bi-axially to produce an expanded fluoropolymer membrane.

In at least one embodiment, the expanded fluoropolymer membrane has a microstructure of substantially only fibrils. The expanded fluoropolymer membrane may be thermally retracted in at least one direction to less than about 90% of the initial, expanded fluoropolymer length. Additionally, the expanded fluoropolymer membrane may be restrained in at least one direction during the thermal retraction. In one embodiment, at least one material may be imbibed into the fluoropolymer membrane prior to, during, or subsequent to retraction.

It is yet another object of the present invention to provide an expanded fluoropolymer membrane that includes serpentine fibrils and at least one other material, which may be a fluoropolymer (*e.g*., fluorinated ethylene propylene), an elastomer, or combinations thereof. It is to be appreciated that the other material may include a fluoroelastomer, which is both a fluoropolymer and an elastomer.

It is a further object of the present invention to provide an expanded fluoropolymer membrane that includes serpentine fibrils and at least one additional material incorporated at least partially into the expanded fluoropolymer membrane. In one or more embodiment, the additional material may be a fluoropolymer or an elastomer. The expanded fluoropolymer membrane may possess a microstructure of substantially only fibrils. Additionally, the expanded fluoropolymer membrane may be thermally retracted in at least one direction.

It is also an object of the present invention to provide an article that includes an expanded fluoropolymer membrane and an elastomer where the membrane has serpentine fibrils and a percent unrecoverable strain energy density less than about 85%. In some embodiments, the expanded fluoropolymer membrane has a percent unrecoverable strain energy density of less than about 80%, less than about 70%, and even less than about 60%.

It is another object of the present invention to provide an expanded fluoropolymer membrane having a microstructure that includes serpentine fibrils that is produced by (1) expanding a dried, extruded fluoropolymer tape in at least one direction to produce an initial expanded fluoropolymer membrane and (2) adding a solvent to the initial expanded fluoropolymer membrane to retract the expanded fluoropolymer membrane in at least one direction of expansion. Additionally, the membrane may be imbibed with an elastomer, a fluoropolymer, a fluoroelastomer, or combinations thereof prior to retraction, during retraction, or subsequent to retraction.

The foregoing and other objects, features, and advantages of the invention will appear more fully hereinafter from a consideration of the detailed description that follows. It is to be expressly understood, however, that the drawings are for illustrative purposes and are not to be construed as defining the limits of the invention.

### BRIEF DESCRIPTION OF THE FIGURES

The advantages of this invention will be apparent upon consideration of the following detailed disclosure of the invention, especially when taken in conjunction with the accompanying drawings wherein:
Figure 1 is a schematic illustration of an exemplary, idealized serpentine fibril;
Figure 2a is a scanning electron micrograph (SEM) of the surface of a prior art precursor membrane;
Figure 2b is a scanning electron micrograph of the surface of an inventive membrane in a retracted state, the membrane having been formed from the precursor membrane shown in Figure 2a;
Figure 2c is a scanning electron micrograph of the surface of the inventive membrane of Figure 2b after subsequent elongation;
Figure 3a is a scanning electron micrograph of the surface of a prior art precursor membrane;
Figure 3b is a scanning electron micrograph of the surface of an inventive membrane in a retracted state, the membrane having been formed from the precursor membrane shown Figure 3a;
Figure 3c is a scanning electron micrograph of the surface of the inventive membrane of Figure 3b after subsequent elongation;
Figure 4a is a scanning electron micrograph of the surface of a prior art precursor membrane;
Figure 4b is a scanning electron micrograph of the surface of an inventive membrane in a retracted state, the membrane having been formed from the precursor membrane of Figure 4a;
Figure 4c is a scanning electron micrograph of the surface of the inventive membrane of Figure 4b after subsequent elongation;
Figure 5a is a scanning electron micrograph of the surface of a composite with the copolymer removed;
Figure 5b is a scanning electron micrograph of the surface of an inventive composite in a retracted state with the copolymer removed, the composite having been formed from the precursor membrane of Figure 5a;
Figure 5c is a scanning electron micrograph of the surface of the inventive composite of Figure 5b with the copolymer removed after subsequent elongation;
Figure 5d is a graphical illustration of tensile stress versus strain of a restrained sample composite in the strongest direction according to one embodiment of the present invention;
Figure 5e is a graphical illustration of tensile stress versus strain of a retracted sample composite in the strongest direction according to one embodiment of the present invention;
Figure 5f is a graphical illustration of tensile stress versus strain of a restrained sample composite in the strongest direction;
Figure 5g is a graphical illustration of stress versus strain of a retracted composite in the strongest direction;
Figure 6a is a scanning electron micrograph of the surface of a prior art precursor membrane;
Figure 6b is a scanning electron micrograph of the surface of an inventive membrane in a retracted state, the membrane having been formed from the precursor membrane of Figure 6a;
Figure 6c is a scanning electron micrograph of the surface of the inventive membrane of Figure 6b after subsequent elongation;
Figure 6d is a graphical illustration of tensile stress versus strain of the precursor membrane orthogonal to the strongest direction;
Figure 6e is a graphical illustration of tensile stress versus strain of a retracted sample membrane orthogonal to the strongest direction;
Figure 7a is a graphical illustration showing the unrecoverable strain energy density of a sample;
Figure 7b is a graphical illustration showing the recoverable strain energy density of the sample of 7a; and
Figure 7c is a graphical illustration showing the total strain energy density of the sample of Figure 7a.

### DETAILED DESCRIPTION OF THE INVENTION

Unless defined otherwise, all technical and scientific terms used herein have the same meaning as commonly understood by one of ordinary skill in the art to which the invention belongs. In the drawings, the thickness of the lines, layers, and regions may be exaggerated for clarity. Like numbers found throughout the figures denote like elements.

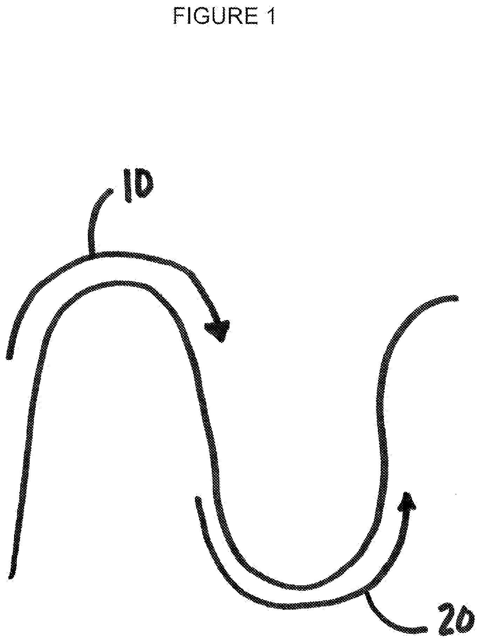

The present invention is directed to fluoropolymer membranes that exhibit high elongation while substantially retaining the strength properties of the fluoropolymer membrane. Such membranes characteristically possess serpentine fibrils, such as the idealized serpentine fibril exemplified in Figure 1. As depicted generally in Figure 1, a serpentine fibril curves or turns generally one way in the direction of arrow 10 then generally another way in the direction of arrow 20. In one embodiment, the fluoropolymer membranes are expandable fluoropolymer membranes. Non-limiting examples of expandable fluoropolymers include, but are not limited to, expanded PTFE, expanded modified PTFE, and expanded copolymers of PTFE. Patents have been filed on expandable blends of PTFE, expandable modified PTFE, and expanded copolymers of PTFE, such as U.S. Patent No. 5,708,044 to Branca; U.S. Patent No. 6,541,589 to Baillie; U.S. Patent No. 7,531,611 to Sabol et al.; U.S. Patent Application No. 11/906,877 to Ford; and U.S. Patent Application No. 12/410,050 to Xu et al.

The high elongation is enabled by forming relatively straight fibrils into serpentine fibrils that substantially straighten upon the application of a force in a direction opposite to the compressed direction. The creation of the serpentine fibrils can be achieved through a thermally-induced controlled retraction of the expanded polytetrafluoroethylene (ePTFE), through wetting the article with a solvent, such as, but not limited to, isopropyl alcohol or Fluorinert® (a perfluorinated solvent commercially available from 3M, Inc., St. Paul, MN), or by a combination of these two techniques. The retraction of the article does not result in visible pleating, folding, or wrinkling of the ePTFE, unlike what occurs during mechanical compression. The retraction also can be applied to very thin membranes, unlike known methods. During the retraction process, the fibrils not only become serpentine in shape but also may also increase in width.

In general, for unrestrained articles, the higher the temperature and the longer the dwell time, the higher the degree of retraction up to the point of maximum retraction. In addition, the speed of retraction can be increased by increasing the retraction temperature.

The precursor materials can be biaxially expanded ePTFE membranes. In one embodiment, materials such as those made in accordance with the general teachings of U.S. Patent No. 7,306,729 to Bacino, et al. are suitable precursor membranes, especially if small pore size articles are desired. These membranes may possess a microstructure of substantially only fibrils. The precursor membrane may or may not be amorphously locked. The precursor membrane may also be at least partially filled, coated, or otherwise combined with additional materials. For example, the precursor membrane may be at least partially coated with fluorinated ethylene propylene.

The precursor membrane may be restrained in one or more directions during the retraction process in order to prescribe the desired amount of elongation of the final article. The amount of elongation is directly related to, and determined by, the amount of retraction. In the instant invention, the amount of retraction can be less than about 90%, 75%, 50% or 25% of the initial un-retracted length. The resultant amounts of elongation in the direction of retraction can be at least about 60%, 80%, 100%, 200%, 300%, 400%, 500%, 600%, or even greater, including any and all percentages therebetween.

The retraction temperature range includes temperatures that result in the retraction of the precursor membrane. In some instances, the retraction temperature can exceed the amorphous locking temperature of the precursor membrane.

In one embodiment, retraction can be achieved in a uniaxial tenter frame by positioning the rails at a distance less than the width of the precursor membrane prior to the application of heat or solvent or both. When using a biaxial tenter frame, one or both of the sets of grips, pins, or other suitable attachment means can similarly be positioned at a distance less than the dimensions of the precursor membrane. It is to be appreciated that these retraction means differ from the mechanical compression taught by the House and Sowinski patents noted above.

In another embodiment, the article can be retracted while being held by hand. A tubular article can be retracted by fitting it over a mandrel prior to retraction. In yet another embodiment, the membrane can be placed in an oven and allowed to retract unrestrained. It is to be understood that any suitable means of retracting the article that does not result in the formation of visible folds, pleats, or wrinkles can be employed. The resulting retracted articles surprisingly exhibit high elongation while substantially retaining the strength properties of the fluoropolymer membrane. Surprisingly, such retracted membranes characteristically possess serpentine fibrils. In certain instances, it may be necessary to partially elongate the retracted membrane in order to observe the serpentine fibrils with magnification.

In another embodiment of the present invention, the precursor membranes described above can be imbibed with an elastomeric material prior, during, or subsequent to retraction to form a composite. In the absence of such elastomeric materials, fluoropolymer articles having serpentine fibrils do not exhibit appreciable recovery after elongation. Suitable elastomeric materials may include, but are not limited to, PMVE-TFE (perfluoromethylvinyl ether-tetrafluoroethylene) copolymers, PAVE-TFE (perfluoro (alkyl vinyl ether)-tetrafluoroethylene) copolymers, silicones, polyurethanes, and the like. It is to be noted that PMVE-TFE and PAVE-TFE are fluoroelastomers. Other fluoroelastomers are suitable elastomeric materials. The resultant retracted article not only possesses high elongation while substantially retaining the strength properties of the fluoropolymer membrane, but also possesses the additional property of low percent unrecoverable strain energy density. These articles can exhibit percent unrecoverable strain energy density values less than about 85%, less than about 80%, less than about 70%, less than about 60%, and lower, including any and all percentages therebetween.

In an alternate embodiment, the ePTFE precursor membrane can be imbibed or coated, at least partially or substantially completely, or otherwise combined with at least one other material that may include, but is not limited to, fluorinated ethylene propylene (FEP), other fluoropolymers, polymers, copolymers, or terpolymers, THV (a terpolymer of tetrafluoroethylene, hexafluoropropylene, and vinylidene fluoride), PFA (perfluoroalkoxy copolymer resin), ECTFE (ethylene chlorotrifluoroethylene), PVDF (polyvinylidene fluoride), and PEEK (polyether ether ketone). The fluoropolymer membrane may be imbibed during, prior, or subsequent to retraction.

Articles of the present invention can take various forms including, but not limited to, sheets, tubes, and laminates.

Having generally described this invention, a further understanding can be obtained by reference to certain specific examples illustrated below which are provided for purposes of illustration only and are not intended to be all inclusive or limiting unless otherwise specified.

### Testing Methods

It should be understood that although certain methods and equipment are described below, any method or equipment determined suitable by one of ordinary skill in the art may be alternatively utilized.

### Mass, Thickness, and Density

Membrane samples were die cut to form rectangular sections about 2.54 cm by about 15.24 cm to measure the weight (using a Mettler-Toledo analytical balance model AG204) and thickness (using a Käfer Fz1000/30 snap gauge). Using these data, density was calculated with the following formula: ρ = m/(w*l*t), in which: ρ = density (g/cm³), m = mass (g), w = width (cm), l = length (cm), and t = thickness (cm). The average of three measurements was reported.

### Matrix Tensile Strength (MTS) of Membranes

Tensile break load was measured using an INSTRON 122 tensile test machine equipped with flat-faced grips and a 0.445 kN load cell. The gauge length was about 5.08 cm and the cross-head speed was about 50.8 cm/min. The sample dimensions were about 2.54 cm by about 15.24 cm. For highest strength measurements, the longer dimension of the sample was oriented in the highest strength direction. For the orthogonal MTS measurements, the larger dimension of the sample was oriented perpendicular to the highest strength direction, Each sample was weighed using a Mettler Toledo Scale Model AG204, then the thickness was measured using the Käfer FZ1000/30 snap gauge; alternatively, any suitable means for measuring thickness may be used. The samples were then tested individually on the tensile tester. Three different sections of each sample were measured. The average of the three maximum loads (*i.e*., peak force) measurements was reported. The longitudinal and transverse matrix tensile strengths (MTS) were calculated using the following equation: MTS= (maximum load/cross-section area)*(bulk density of PTFE)/ (density of the porous membrane), where the bulk density of the PTFE was taken to be about 2.2 g/cm³.

### Tensile Strength of Composites

Composite tensile testing was performed using an RSA3 dynamic mechanical analyzer (TA Instruments, New Castle, DE) with a 3500 g load cell. 13 mm x 39 mm rectangular samples were mounted with a 20 mm gauge length and strained at a rate of 1000%/minute. For highest strength measurements, the longer dimension of the sample was oriented in the highest strength direction. For the orthogonal tensile strength measurements, the larger dimension of the sample was oriented perpendicular to the highest strength direction. Reported data are an average of at least 3 measurements.

### Elongation Testing

Elongation of the retracted article can be measured by any suitable application of tensile force, such as, for example, by the use of a tensile testing machine, by hand, or by applying internal pressure to a tubular article. In the instant invention, elongation was performed at a rate of about 10% per second in all directions that were elongated. Elongation was calculated as the final length minus the initial length, divided by the initial length, and was reported as a percentage.

### Gurley Number

Gurley number refers to the time in seconds for 100 cc of air to flow through a 6.45 cm² sample at 124 mm of water pressure. The samples were measured in a Genuine Gurley Densometer Model 4340 Automatic Densometer. The reported value represents the average measurement of at least 3 samples.

### Percent Unrecoverable Strain Energy Density

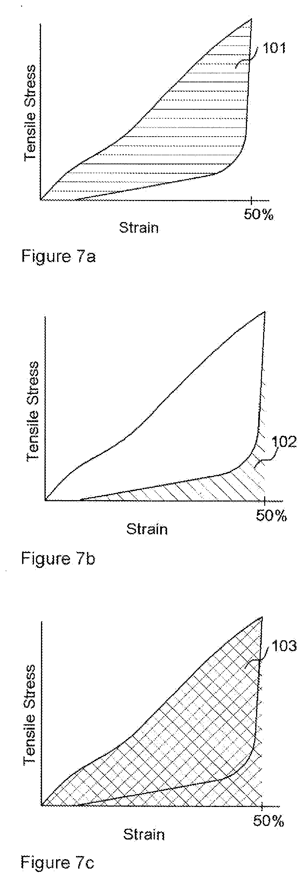

The percent unrecoverable strain energy density of composites was measured using an RSA3 dynamic mechanical analyzer (TA Instruments, New Castle, DE) with a 3500 g load cell. A 13 mm x 39 mm rectangular sample was cut so that the longer dimension was oriented in the highest strength direction. The sample was mounted in film/fiber tension grips with a 20 mm gauge length. The grips were programmed to elongate the sample to 50% strain at a rate of 200 mm/minute and were then immediately returned to the initial displacement at a rate of 200 mm/minute. Load and displacement values were collected, converted to stress and strain values, and then graphed. The unrecoverable strain energy density is represented by the area 101 between the elongation and return curve as depicted in Figure 7a. The recoverable strain energy density is represented by the area 102 in Figure 7b.

The percent unrecoverable strain energy density of the sample is defined by the area 101 between the elongation and return curve as shown in Figure 7a, divided by the crosshatched area 103 under the elongation curve from 0% to 50% strain as shown in Figure 7c, then multiplied by 100%. Reported data are an average of at least 3 measurements.

Should the sample break prior to 50% strain, then another sample should be tested at 50% of the breakage strain to calculate the unrecoverable strain energy density. For samples that are too small to accommodate the 20 mm grip separation and allow enough material within the grips to prevent slippage of the sample within the grips, other combinations of crosshead speed and grip separation may be used provided the ratio of crosshead speed to initial grip separation is equal to 10 minutes⁻¹.

### Scanning Electron Microscopy

Scanning electron micrographs were created choosing magnifications suitable for identifying fibrils. Articles that have been retracted in accordance with the teachings of invention may require elongation in the direction of retraction in order to identify the serpentine fibrils.

### EXAMPLES

### EXAMPLE 1

### Precursor Membrane

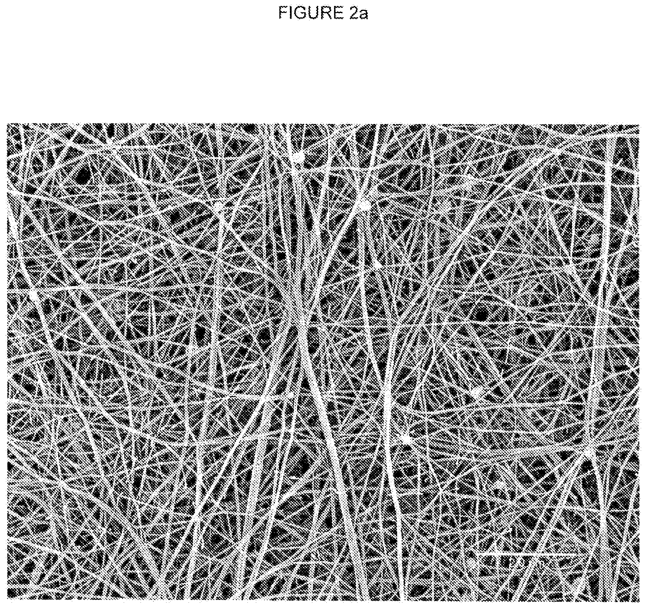

A biaxially expanded PTFE membrane that had not been amorphously locked having the following properties was obtained: thickness = 0.0017 mm, density = 1.58 g/cc, Gurley = 8.8 sec, matrix tensile strength in the strongest direction = 346 MPa; matrix tensile strength in the direction orthogonal to the strongest direction = 303 MPa, elongation at maximum load in the strongest direction = 76.6%, and elongation at maximum load in the direction orthogonal to the strongest direction = 98.6%. As used herein, the phrase "strongest direction" refers to the strongest direction of the precursor membrane. The fibrils of the membrane were substantially straight and the membrane contained substantially only fibrils, as shown in Figure 2a, a scanning electron micrograph (SEM) of the surface of the membrane taken at 10,000x magnification. The precursor membrane and the initial, expanded fluoropolymer membrane may be used interchangeably herein.

### Retracted Membrane

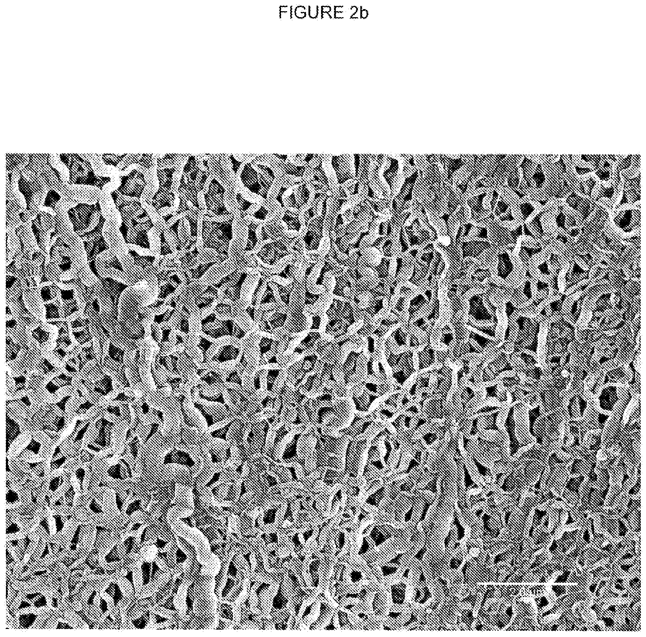

The precursor membrane was cut to dimensions of approximately 500 mm x 500 mm. A 400 mm x 400 mm square was drawn onto the membrane using a felt tip pen and the membrane was clipped at its four corners and hung loosely from a shelf in an oven set to 310 °C. After about five minutes, the now heat-shrunk membrane was removed from the oven. The retracted length dimensions of the square markings were measured to be 194 mm in the strongest direction and 167 mm in the direction orthogonal to the strongest direction. That is, the amount of shrinkage was about 49% of the original length (*i.e*., (194/400) (100%)) in the strongest direction and 42% in the direction orthogonal to the strongest direction. The retracted membrane had the following properties: thickness = 0.0062 mm, density = 2.00 g/cc, Gurley = 527 sec, matrix tensile strength in the strongest direction = 164 MPa, matrix tensile strength in the direction orthogonal to the strongest direction = 124 MPa, elongation at maximum load in strongest direction = 235%, and elongation at maximum load in the direction orthogonal to the strongest direction = 346%. The fibrils of the membrane had become serpentine in shape as shown in Figure 2b, a SEM of the surface of the membrane taken at 10,000x magnification.

### Elongated Retracted Membrane

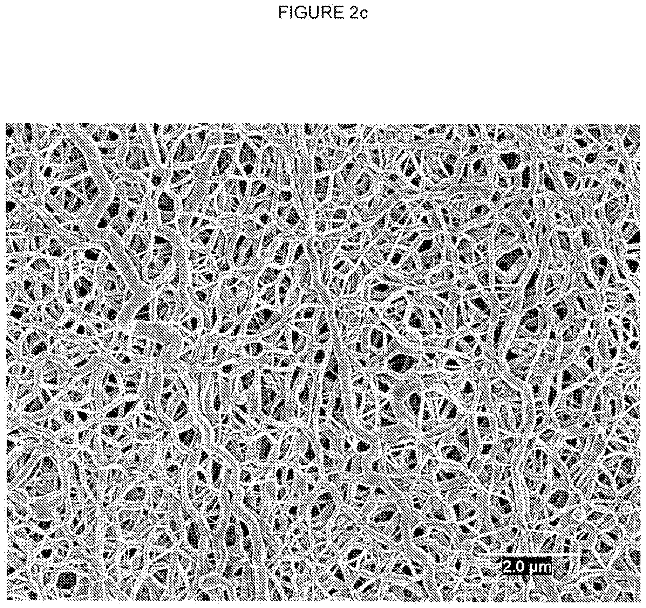

A length of the retracted membrane was stretched by hand to about 57% of the original precursor membrane in the strongest direction and to about 50% of the original precursor membrane in the direction orthogonal to the strongest direction. The fibrils still had a serpentine shape after elongation, as shown in Figure 2c, a SEM of the surface of the membrane taken at 10,000x magnification.

### EXAMPLE 2

### Precursor Membrane

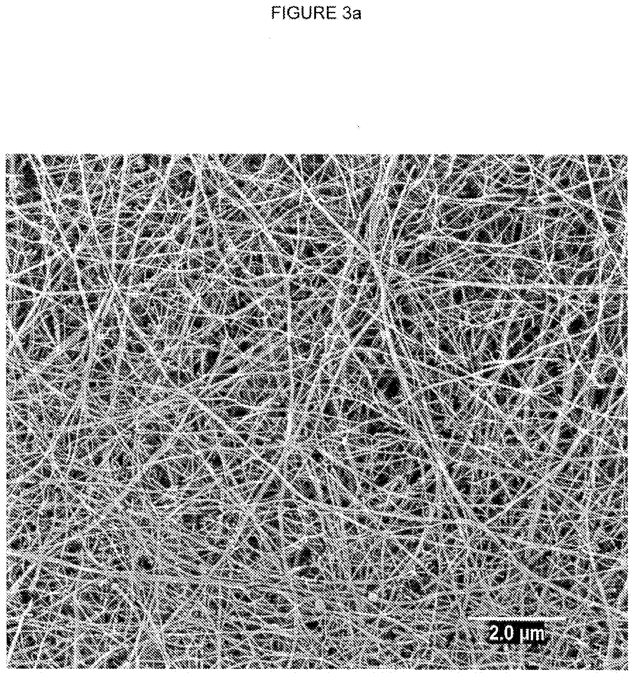

A biaxially expanded PTFE membrane that had not been amorphously locked having the following properties was obtained: thickness = 0.00051 mm, density = 2.00 g/cc, Gurley = 3.1 sec, matrix tensile strength in the strongest direction = 500 MPa. The matrix tensile strength in the direction orthogonal to the strongest direction = 324 MPa, elongation at maximum load in the strongest direction = 68.3%, and elongation at maximum load in the direction orthogonal to the strongest direction = 87.7%. The fibrils of the membrane were substantially straight as shown in Figure 3a, a SEM of the surface of the membrane taken at 10,000x magnification.

### Example 2a:

### Retracted Membrane

A roll of precursor membrane, wherein the length direction corresponded with the strongest direction of the membrane, was restrained in the clamps of a heated, uniaxial tenter frame and fed into the heated chamber of the tenter frame. The oven temperature was set to about 280 °C. The rails of the tenter frame within the heated chamber were angled inward in order to allow membrane shrinkage to about 54% of its original width in response to the heat. The line speed was set to provide a dwell time of about two minutes within the heated chamber.

The initial and final widths of the membrane were 1572 mm and 848 mm, respectively. The retracted membrane had the following properties: thickness = .00152 mm, density = 1.1 g/cc, Gurley = 2.8 sec, matrix tensile strength in the strongest direction = 517 MPa, matrix tensile strength in the direction orthogonal to the strongest direction = 160 MPa, elongation at maximum load in strongest direction = 63%, and elongation at maximum load in the direction orthogonal to the strongest direction = 188%.

### Example 2b:

### Retracted Membrane

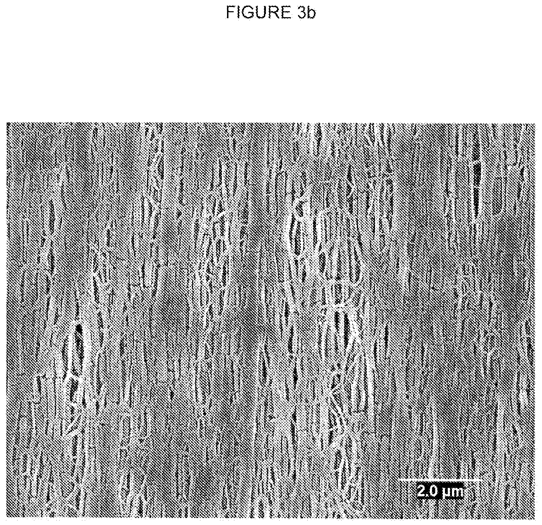

A roll of the precursor membrane, where the length direction corresponded with the strongest direction of the membrane, was fed into a heated, uniaxial tenter frame as described in Example 2a with the exception that the membrane shrinkage was performed to about 23% of the original width of the membrane. The initial and final widths of the membrane were 1572 mm and 353 mm, respectively. The retracted membrane had the following properties: thickness = 0.00406 mm, density = 1.3 g/cc, Gurley = 88.9 sec, matrix tensile strength in the strongest direction = 530 MPa, matrix tensile strength in the direction orthogonal to the strongest direction = 52 MPa, elongation at maximum load in the strongest direction = 49.1%, and elongation at maximum load in the direction orthogonal to the strongest direction = 665%. A SEM of the surface of the membrane was taken at 10,000x magnification and is shown in Figure 3b.

### Elongated Retracted Membrane

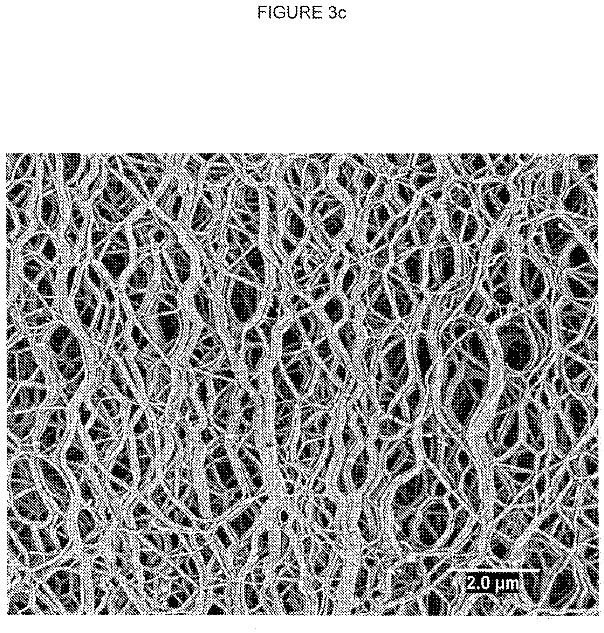

A length of the retracted membrane was stretched by hand to about 35% of the original precursor membrane width in the direction orthogonal to the strongest direction. The fibrils were seen to have a serpentine shape after elongation as indicated in Figure 3c, a SEM of the surface of the membrane taken at 10,000x magnification.

### EXAMPLE 3

### Precursor Membrane

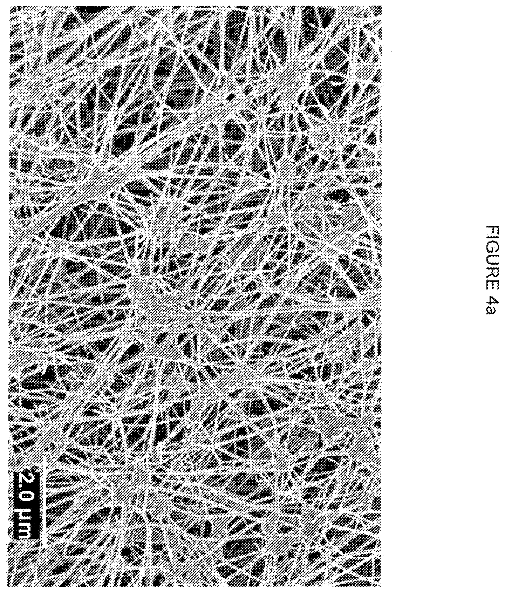

An expanded PTFE membrane that had been amorphously locked and had the following properties was obtained: thickness = 010 mm, density = 507 g/cc, Gurley = 5.5 sec, matrix tensile strength in the strongest direction = 96 MPa, matrix tensile strength in the direction orthogonal to the strongest direction = 55 MPa, elongation at maximum load in strongest direction = 33%, and elongation at maximum load in direction orthogonal to the strongest direction = 59.2%. The fibrils of the membrane were substantially straight as shown in Figure 4a, a SEM of the surface of the membrane taken at 10,000x magnification.

### Retracted Membrane

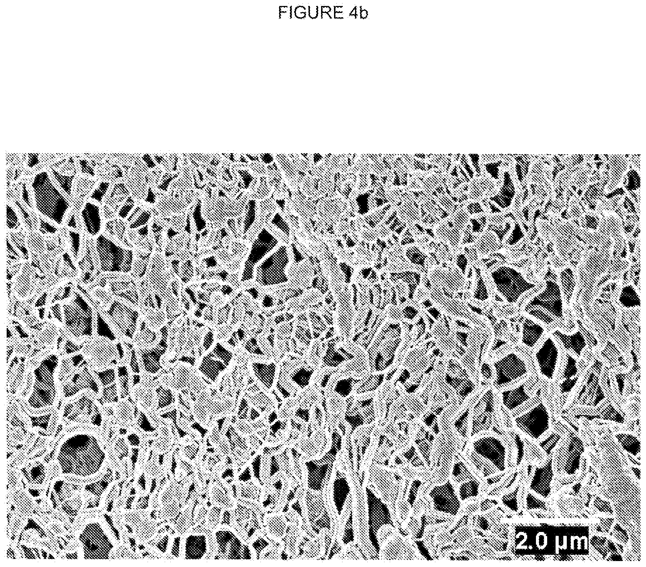

The precursor membrane was cut to dimensions of approximately 200 mm x 200 mm. An array of 10 mm squares were drawn onto the membrane using a felt tip pen and the membrane was clipped at four corners and hung loosely in an oven set to 200 °C. After about 20 minutes, the heat-shrunk membrane was removed from the oven. The retracted dimensions of the square markings were measured and it was determined that the membrane shrunk to about 72% in the strongest direction and to about 67% in the direction orthogonal to the strongest direction. A SEM of the surface of the retracted membrane taken at 10,000x magnification is shown in Figure 4b.

### Elongated Retracted Membrane

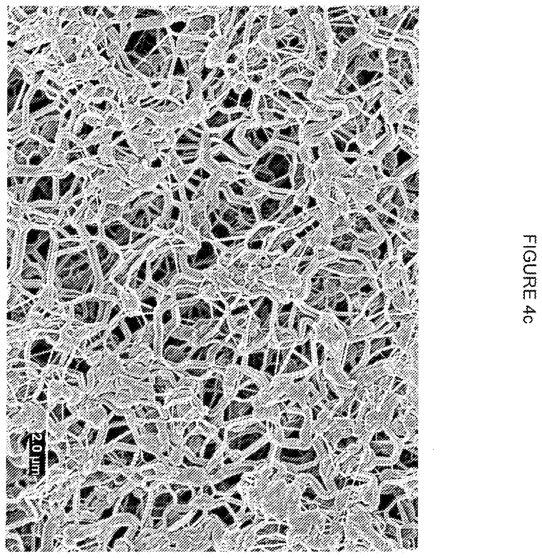

A length of the retracted membrane was stretched by hand to about 74% of the original precursor membrane length in the strongest direction and to about 70% of the original precursor membrane length in the direction orthogonal to the strongest direction. The fibrils were seen to have a serpentine shape after elongation as depicted in Figure 4c, a SEM of the surface of the membrane taken at 10,000x magnification.

### EXAMPLE 4

### Precursor Membrane

A biaxially expanded ePTFE membrane that had not been amorphously locked and had the following properties was obtained: thickness = 0.0023 mm, density = 0.958 g/cc, matrix tensile strength in the strongest direction = 433 MPa, matrix tensile strength in the direction orthogonal to the strongest direction = 340 MPa, elongation at maximum load in the strongest direction = 39%, and elongation at maximum load in the direction orthogonal to the strongest direction = 73%. Upon tensioning by hand, the membrane did not noticeably retract upon the release of the tension.

### Restrained Composite

A copolymer comprising tetrafluoroethylene (TFE) and perfluoro(methyl vinylether) (PMVE) as described in U.S. Patent No. 7,049,380 to Chang, et al. was obtained with a PMVE/TFE ratio of 2:1. This copolymer was dissolved in a fluorinated solvent (Fluorinert Electronic Liquid FC-72, 3M Inc., St. Paul, MN) in a ratio of 3 parts copolymer to 97 parts solvent by weight. A continuous slot die coating process operating at a line speed of approximately 1.8 m/min and a solution coating rate of approximately 96 g/min was utilized to imbibe this solution into the ePTFE precursor membrane that was fed from a roll. The imbibed ePTFE membrane was restrained in the clamps of a heated, uniaxial tenter frame. The imbibed membrane was fed into a tenter frame where the length direction corresponded with the direction orthogonal to the strongest direction of the membrane. The precursor membrane was fed into a 2.4 m long heated chamber of the tenter frame. The rails of the tenter frame were substantially parallel within the heated chamber, resulting in a minimal retraction of the imbibed membrane as it was heated and the fluorinated solvent was driven off. The line speed was set to provide a dwell time of about 45 seconds within the heated chamber and the material reached a maximum temperature of approximately 180°C.

This imbibing process enabled the copolymer to penetrate the pores of the membrane as well as to create a coating of the copolymer on the surface of the membrane, thereby creating a restrained composite. The restrained composite had the following properties: thickness = 0.0152 mm, maximum tensile stress in the strongest direction = 34.4 MPa, maximum tensile stress in the direction orthogonal to the strongest direction = 68.9 MPa, elongation at maximum load in the strongest direction = 119%, and elongation at maximum load in the direction orthogonal to the strongest direction = 39%.

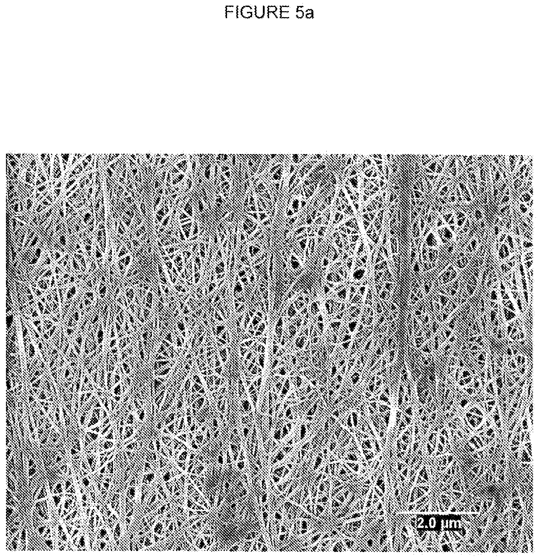

The copolymer component of a restrained sample was removed to enable SEM imaging of the ePTFE structure. The removal process was performed as follows. A 45 mm circular sample of each composite was restrained using a 35 mm diameter plastic hoop. The samples were submerged in 95 g of Fluorinert Electronic Liquid FC-72 (3M Inc., St. Paul, MN) and allowed to soak without agitation. After approximately one hour, the fluorinated solvent was poured off and replaced with 95 g of fresh solvent. This process was repeated for a total of 5 soaking cycles, the first 4 cycles for approximately 1 hour, and the 5th cycle for approximately 24 hours. The restrained composite with the copolymer removed is shown in Figure 5a (a SEM of the surface of the membrane taken at 10,000 x magnification).

### Retracted Composite

A retracted composite was made in accordance with the same process as was used to create the restrained composite with the exception that the rails of the tenter frame were not oriented parallel to one another. The rails were positioned to accommodate a 100 mm wide imbibed ePTFE membrane entering the heated chamber, enabling the heated composite to shrink due to the application of heat so that it exited the chamber with a 56 mm width.

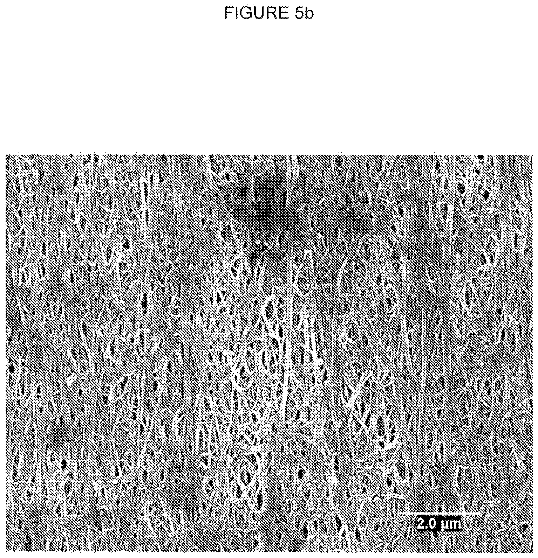

The retracted composite had the following properties: thickness = 0.0165 mm, maximum stress in the strongest direction = 26.3 MPa, maximum stress in the direction orthogonal to the strongest direction = 53.9 MPa, elongation at maximum load in the strongest direction = 170%, and elongation at maximum load in the direction orthogonal to the strongest direction = 55%. The retracted composite with the copolymer removed, in the manner described above, is shown in Figure 5b (a SEM of the surface of the membrane taken at 10,000x magnification).

### Elongated Retracted Membrane

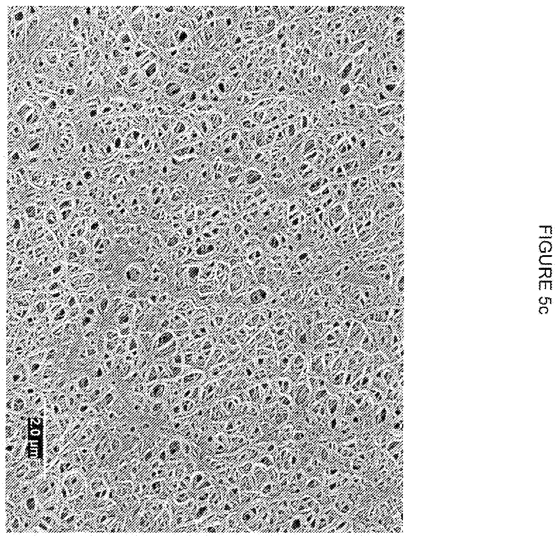

A portion of the retracted composite with the copolymer removed was stretched by hand to about 76% of the original precursor membrane in the direction orthogonal to the strongest direction. The fibrils were noted to have a serpentine shape as shown in Figure 5c, a SEM of the surface of the retracted composite with the copolymer removed taken at 10,000x magnification.

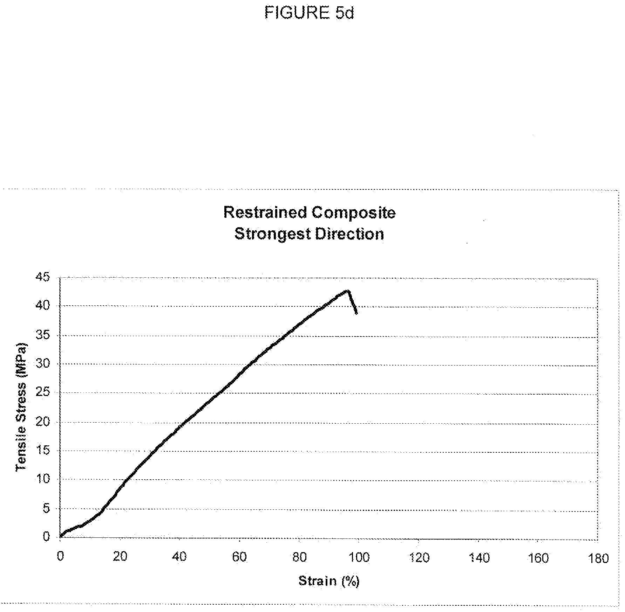

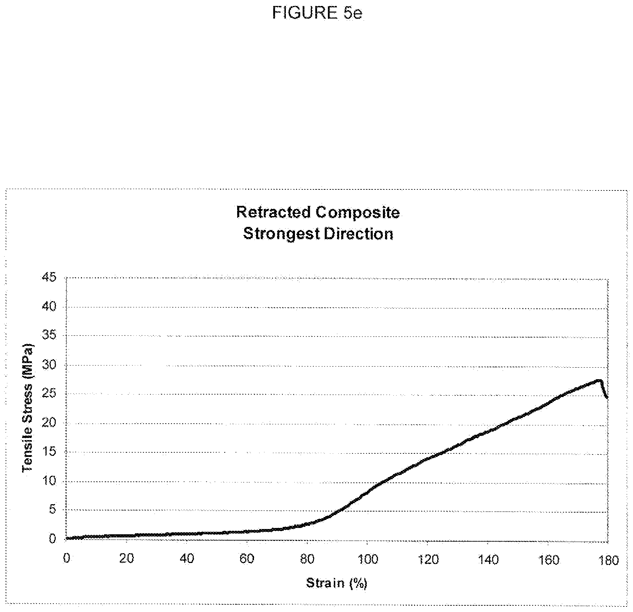

Figures 5d and 5e are tensile stress versus strain curves corresponding to a sample of each of the copolymer-containing restrained and copolymer-containing retracted composites, respectively, of Example 4. The tensile tests were performed in accordance with the above-described test methods. The curve for the restrained composite sample exhibited a relatively constant modulus (*i.e*., the slope of the tensile stress versus strain curve) throughout the tensile test. The curve for the retracted composite sample, on the other hand, exhibited a much lower, relatively constant modulus for strains up to about 80%. The retracted composite curve also exhibited a much higher and relatively constant modulus past about 80% strain up until failure (*i.e*. about 180% strain).

Therefore, the retracted composite can be elongated at a much lower tensile stress than the restrained composite until reaching the amount of strain where the slope of the curve substantially increases. Furthermore, the strains corresponding to failure for the restrained and retracted composites were about 120% and about 180%, respectively.

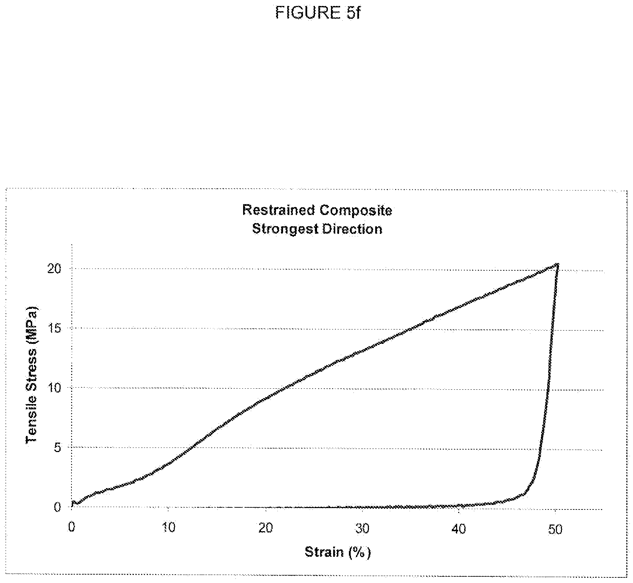

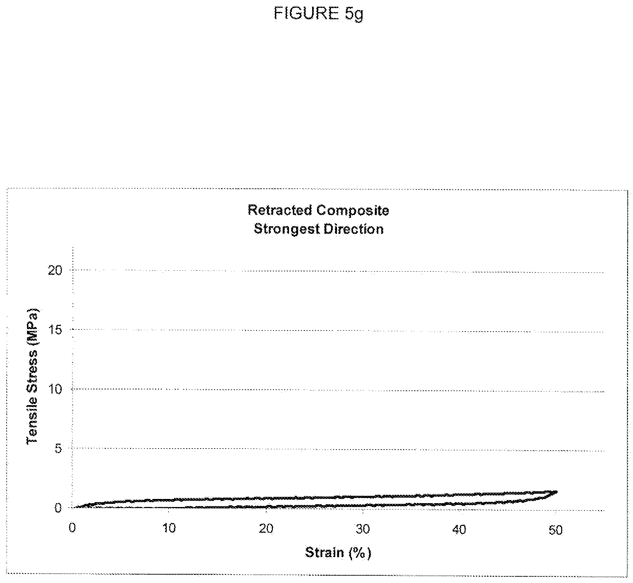

Figures 5f and 5g are tensile stress versus strain curves corresponding to a sample of each of the copolymer-containing restrained and copolymer-containing retracted composites, respectively, of Example 4. The percent unrecoverable strain energy density tests were performed in accordance with the above-described test method.

The unrecoverable strain energy density of the restrained composite was large and is depicted by the area bound by the elongation and return curves in Figure 5f. In comparison, the unrecoverable strain energy density of the retracted composite was much smaller, as depicted by the area bound by the elongation and return curves in Figure 5g. The percent unrecoverable strain energy density of the restrained composite was about 94.0% and the percent unrecoverable strain energy density of the retracted composite was about 67.9%.

### EXAMPLE 5

### Precursor Membrane

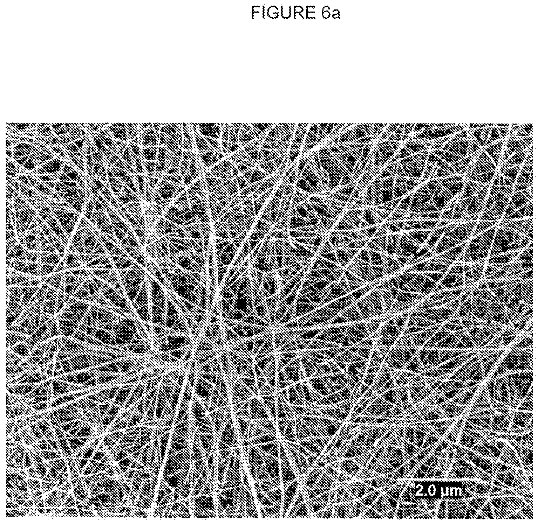

An amorphously locked biaxially expanded ePTFE membrane having the following properties was obtained: thickness = 0.00254 mm, density = 0.419 g/cc, Gurley = 4.3 sec, matrix tensile strength in the strongest direction = 327 MPa, matrix tensile strength in the direction orthogonal to the strongest direction = 285 MPa, elongation at maximum load in the strongest direction = 42%, and elongation at maximum load in the direction orthogonal to the strongest direction = 21%. The fibrils of the membrane were substantially straight as shown in Figure 6a, a SEM of the surface of the membrane taken at 10,000x magnification.

### Retracted Membrane

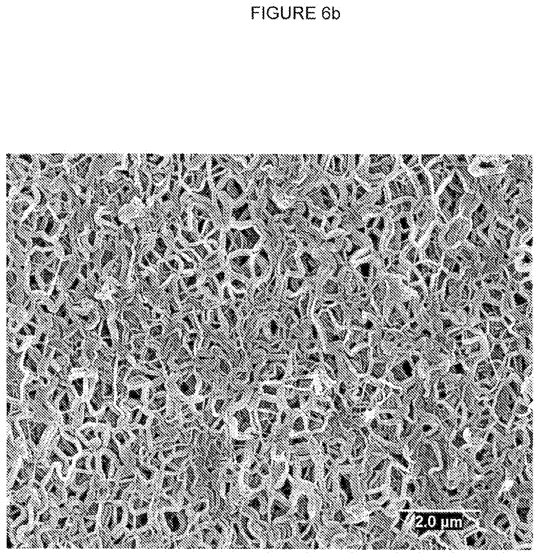

The precursor membrane was biaxially retracted in the same manner as described in Example 1. The resulting retracted membrane was about 51% of the original length of the precursor membrane in the strongest direction and about 37% of the original length of the precursor membrane in the direction orthogonal to the strongest direction. The retracted membrane had the following properties: thickness = 0.00508 mm, density = 1.07 g/cc, Gurley = 33.4 sec, matrix tensile strength in the strongest direction = 169 MPa, matrix tensile strength in the direction orthogonal to the strongest direction = 105 MPa, elongation at maximum load in the strongest direction = 144%, and elongation at maximum load in the direction orthogonal to the strongest direction = 193%. As shown in Figure 6b, a SEM of the surface of the membrane taken at 10,000x magnification, the fibrils of the membrane had become serpentine in shape.

### Elongated Retracted Membrane

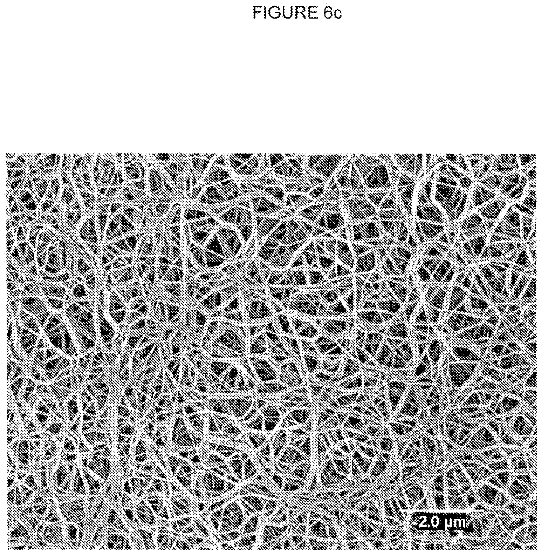

A 100 mm by 100 mm portion of the retracted membrane was subsequently elongated at ambient temperature in a tenter frame capable of biaxial stretching. The degree of elongation was selected to return the membrane to about 93% of its original length in the strongest direction and to about 68% of its original length in the direction orthogonal to the strongest direction. The membrane was elongated simultaneously in both directions. The dimensions of the elongated portion of the retracted membrane were about 224 mm by 224 mm. The elongated membrane had the following properties: thickness = 0.00508 mm, density = 0.445 g/cc, and Gurley = 7.33 sec. The fibrils of the membrane were serpentine in shape as shown in Figure 6c, a SEM of the surface of the membrane taken at 10,000x magnification.

Samples of the elongated retracted membrane were also tensile tested. The following results were obtained: matrix tensile strength in the strongest direction = 292 MPa, matrix tensile strength in the direction orthogonal to the strongest direction = 221 MPa. The elongation at maximum load in the strongest direction = 98%, and elongation at maximum load in the direction orthogonal to the strongest direction = 91%.

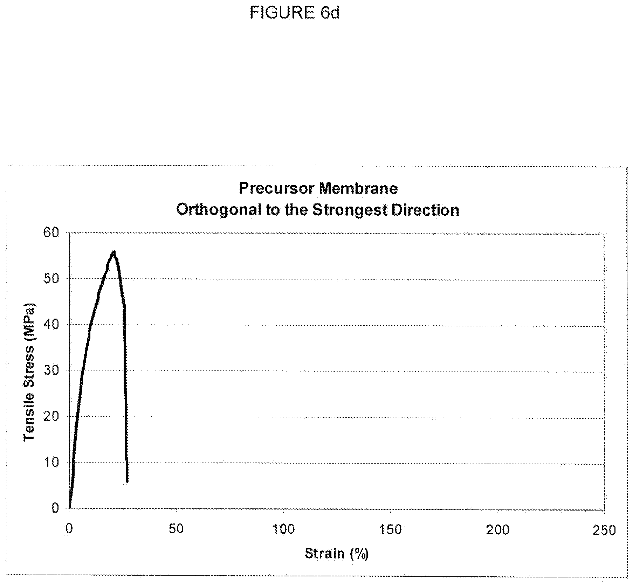

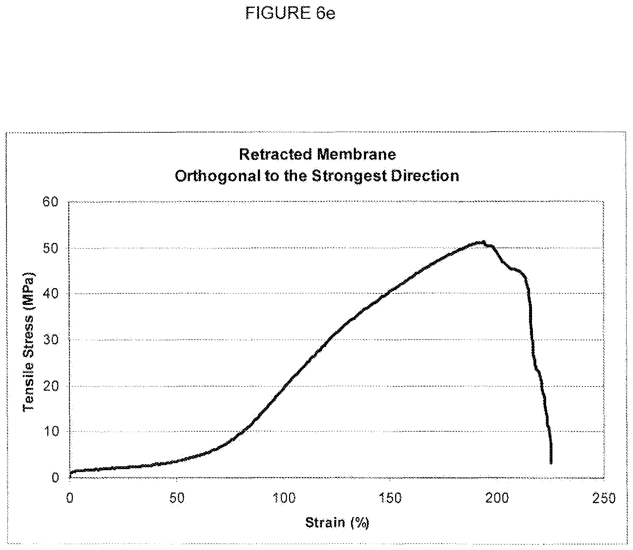

Figures 6d and 6e are stress versus strain curves corresponding to a sample of the restrained and retracted membranes, respectively, of Example 5. The tensile tests were performed in accordance with the above-described test methods. The curve for the precursor membrane exhibited a relatively high and constant modulus (*i.e*., the slope of the stress versus strain curve) up to a strain of about 25%. In contrast, the curve for the retracted membrane sample exhibited a low, relatively constant modulus for strains up to about 80% and then an increased and relatively constant modulus for strains up to about 200%

A summary of the data collected and obtained from Examples 1-5 is set forth in Table 1.

**TABLE 1**

| **Example** | | **Thickness (mm)** | **Density (g/cc)** | **MTS [MPa]/Elong [%] Strongest Direction** | **MTS [MPa]/Elong [%] Orthogonal Direction** | **Gurley (sec)** | **% Unrecoverable Strain Energy Density (%)** |
|---|---|---|---|---|---|---|---|
| **1** | Precursor Membrane | 0.0017 | 1.58 | 346/76.6 | 303/98.6 | 8.8 | n/a |
| | Retracted Membrane | 0.0062 | 2.00 | 164/235 | 124/346 | 527 | n/a |
| | Elongated Retracted Membrane | n/a | n/a | n/a | n/a | n/a | n/a |
| | | | | | | | |
| **2a** | Precursor Membrane | 0.00051 | 2.00 | 500/68.3 | 324/87.7 | 3.1 | n/a |
| | Retracted Membrane | 0.00152 | 1.10 | 517/63 | 160/188 | 2.8 | n/a |
| | | | | | | | |
| **2b** | Precursor Membrane | 0.00051 | 2.00 | 500/68.3 | 324/87.7 | 3.1 | n/a |
| | Retracted Membrane | 0.00406 | 1.30 | 530/49.1 | 52/665 | 88.9 | n/a |
| | Elongated Retracted Membrane | n/a | n/a | n/a | n/a | n/a | n/a |
| **3** | Precursor Membrane | 0.01 | 0.51 | 96/33 | 55/59.2 | 5.5 | n/a |
| | Retracted Membrane | n/a | n/a | n/a | n/a | n/a | n/a |
| | Elongated Retracted Membrane | n/a | n/a | n/a | n/a | n/a | n/a |
| | | | | | | | |
| **4** | Precursor Membrane | 0.0023 | 0.96 | 433/39 | 340/73 | n/a | n/a |
| | Copolymer-Containing Restrained Composite | 0.0152 | n/a | 34.4*/119 | 68.9*/39 | n/a | 94 |
| | Copolymer-Containing Retracted Composite | 0.0165 | n/a | 26.3/170 | 53.9/55 | n/a | 67.9 |
| | | | | | | | |
| **5** | Precursor Membrane | 0.00254 | 0.419 | 327/42 | 285/21 | 4.3 | n/a |
| | Retracted Membrane | 0.0051 | 1.07 | 169/144 | 105/193 | 33.4 | n/a |
| | Elongated Retracted Membrane | 0.0051 | 0.45 | 292/98 | 221/91 | 7.33 | n/a |

| | | | | | | | |
|---|---|---|---|---|---|---|---|
| * Maximum Tensile Strength (MPa) | | | | | | | |

## Claims

1. An article comprising a retracted expanded fluoropolymer membrane having a microstructure including serpentine fibrils, the serpentine fibrils curving in a first direction and then a second direction, the serpentine fibrils being produced by the process comprising:
a. expanding a dried, extruded fluoropolymer tape in at least one direction to produce an initial expanded fluoropolymer membrane; and either
b. heating the initial expanded fluoropolymer membrane to thermally retract the expanded fluoropolymer membrane in at last one direction of expansion, or
c. adding a solvent to the initial expanded fluoropolymer membrane to retract the expanded fluoropolymer membrane in at least one direction of expansion.

2. The article of claim 1, wherein the fluoropolymer comprises polytetrafluoroethylene.

3. The article of claim 1 when the initial expanded fluoropolymer membrane is thermally retracted, wherein the retracted expanded fluoropolymer membrane is thermally retracted in at least one direction to less than about 90%, less than about 75%, less than about 50% or less than about 25% of the initial, expanded fluoropolymer membrane length.

4. An article according to claim 1, further comprising at least one additional material.

5. The article of claim 4, wherein the additional material is selected from the group consisting of a fluoropolymer, an elastomer and combinations thereof, optionally wherein the fluoropolymer is fluorinated ethylene propylene.

6. An article according to claim 1, further comprising at least one additional material incorporated at least partially into the retracted expanded fluoropolymer membrane, optionally wherein the at least one additional material comprises a fluoropolymer or an elastomer.

7. The article of claim 1 when the initial expanded fluoropolymer membrane is thermally retracted wherein the retracted expanded fluoropolymer membrane has a microstructure of substantially only fibrils.

8. The article of claim 6, wherein the retracted expanded fluoropolymer membrane is thermally retracted in at least one direction, optionally wherein the expanded fluoropolymer membrane is restrained in at least one direction during said thermal retraction.

9. The article of claim 4 or 1 when the initial expanded fluoropolymer membrane is retracted with solvent, wherein at least one material is imbibed into said expanded fluoropolymer membrane prior to retracting said expanded fluoropolymer membrane, as said expanded fluoropolymer membrane is retracted or subsequent to retracting said expanded fluoropolymer membrane,
optionally wherein said at least one material is selected from the group consisting of a fluoropolymer, an elastomer and combinations thereof.

## Patentansprüche

1. Artikel, der eine geschrumpfte expandierte Fluorpolymermembran umfasst, die eine Mikrostruktur aufweist, die schlangenförmige Fibrillen beinhaltet, wobei sich die schlangenförmigen Fibrillen in eine erste Richtung und dann in eine zweite Richtung biegen, wobei die schlangenförmigen Fibrillen durch das Verfahren hergestellt werden, das Folgendes umfasst:
a. Ausdehnen eines getrockneten, extrudierten Fluorpolymerbands in mindestens eine Richtung, um eine ursprüngliche expandierte Fluorpolymermembran herzustellen; und entweder
b. Erhitzen der ursprünglichen expandierten Fluorpolymermembran, um die expandierte Fluorpolymermembran in mindestens eine Ausdehnungsrichtung thermisch zu schrumpfen; oder
c. Zugeben eines Lösungsmittels zu der ursprünglichen expandierten Fluorpolymermembran, um die expandierte Fluorpolymermembran in mindestens eine Ausdehnungsrichtung zu schrumpfen.

2. Artikel nach Anspruch 1, wobei das Fluorpolymer Polytetrafluorethylen umfasst.

3. Artikel nach Anspruch 1, wenn die ursprüngliche expandierte Fluorpolymermembran thermisch geschrumpft ist, wobei die geschrumpfte expandierte Fluorpolymermembran in mindestens eine Richtung weniger als etwa 90 %, weniger als etwa 75 %, weniger als etwa 50 % oder weniger als etwa 25 % der ursprünglichen, expandierten Länge der Fluorpolymermembran thermisch geschrumpft ist.

4. Artikel nach Anspruch 1, der ferner mindestens ein zusätzliches Material umfasst.

5. Artikel nach Anspruch 4, wobei das zusätzliche Material ausgewählt ist aus der Gruppe bestehend aus einem Fluorpolymer, einem Elastomer und Kombinationen daraus, wobei optional das Fluorpolymer fluoriertes Ethylenpropylen ist.

6. Artikel nach Anspruch 1, der ferner mindestens ein zusätzliches Material umfasst, das mindestens teilweise in die geschrumpfte expandierte Fluorpolymermembran integriert ist, wobei optional das mindestens eine zusätzliche Material ein Fluorpolymer oder ein Elastomer umfasst.

7. Artikel nach Anspruch 1, wenn die ursprüngliche expandierte Fluorpolymermembran thermisch geschrumpft ist, wobei die geschrumpfte expandierte Fluorpolymermembran eine Mikrostruktur von im Wesentlichen nur Fibrillen aufweist.

8. Artikel nach Anspruch 6, wobei die geschrumpfte expandierte Fluorpolymermembran in mindestens eine Richtung thermisch geschrumpft ist, wobei optional die expandierte Fluorpolymermembran in mindestens eine Richtung während des thermischen Schrumpfens zurückgehalten wird.

9. Artikel nach Anspruch 4 oder 1, wobei die ursprüngliche expandierte Fluorpolymermembran mit einem Lösungsmittel geschrumpft wird, wobei mindestens ein Material vor dem Schrumpfen der expandierten Fluorpolymermembran in die expandierte Fluorpolymermembran getränkt wird, während die expandierte Fluorpolymermembran geschrumpft wird, oder nach dem Schrumpfen der expandierten Fluorpolymermembran,
wobei optional das mindestens eine Material ausgewählt ist aus der Gruppe bestehend aus einem Fluorpolymer, einem Elastomer und Kombinationen daraus.

## Revendications

1. Article comprenant une membrane en fluoropolymère expansé rétractée ayant une microstructure comprenant des fibrilles serpentines, lesdites fibrilles serpentines étant courbées dans une première direction puis dans une seconde direction, lesdites fibrilles serpentines étant produites par le procédé comprenant :
a. l'expansion d'un ruban en fluoropolymère extrudé séché dans au moins une direction pour produire une membrane en fluoropolymère expansé initiale ; et soit
b. le chauffage de la membrane en fluoropolymère expansé initiale afin de thermorétracter ladite membrane en fluoropolymère expansé dans au moins une direction d'expansion, soit
c. l'ajout d'un solvant à la membrane en fluoropolymère expansé initiale afin de rétracter ladite membrane en fluoropolymère expansé dans au moins une direction d'expansion.

2. Article selon la revendication 1, dans lequel le fluoropolymère comprend du polytétrafluoroéthylène.

3. Article selon la revendication 1 quand la membrane en fluoropolymère expansé initiale est thermorétractée, ladite membrane en fluoropolymère expansé rétractée étant thermorétractée dans au moins une direction à moins d'environ 90 %, à moins d'environ 75 %, à moins d'environ 50 % ou à moins d'environ 25 % de la longueur de la membrane en fluoropolymère expansé initiale.

4. Article selon la revendication 1, comprenant en outre au moins un matériau additionnel.

5. Article selon la revendication 4, dans lequel le matériau additionnel est sélectionné dans le groupe constitué d'un fluoropolymère, un élastomère et de combinaisons de ceux-ci, le fluoropolymère étant éventuellement de l'éthylène-propylène fluoré.

6. Article selon la revendication 1, comprenant en outre au moins un matériau additionnel incorporé au moins partiellement dans la membrane en fluoropolymère expansé rétractée, ledit ou lesdits matériaux additionnels comprenant éventuellement un fluoropolymère ou un élastomère.

7. Article selon la revendication 1 quand la membrane en fluoropolymère expansé initiale est thermorétractée dans lequel la membrane en fluoropolymère expansé rétractée a une microstructure sensiblement constituée seulement de fibrilles.

8. Article selon la revendication 6, dans lequel la membrane en fluoropolymère expansé rétractée est thermorétractée dans au moins une direction, éventuellement dans lequel la membrane en fluoropolymère expansé est retenue dans au moins une direction durant ladite thermorétraction.

9. Article selon la revendication 4 ou 1 quand la membrane en fluoropolymère expansé initiale est rétractée avec du solvant, dans lequel au moins un matériau s'imbibe dans ladite membrane en fluoropolymère expansé avant la rétraction de ladite membrane en fluoropolymère expansé, pendant la rétraction de ladite membrane en fluoropolymère expansé ou après la rétraction de ladite membrane en fluoropolymère expansé,
éventuellement dans lequel le ou les matériaux sont sélectionnés dans le groupe constitué d'un fluoropolymère, d'un élastomère et de combinaisons de ceux-ci.
